# EUROPEAN PATENT APPLICATION

(11) **EP 2 056 114 A1**
(43) Date of publication of application: **06.05.2009**
(21) Application number: 07119462.5
(22) Date of filing: 29.10.2007
(51) Int. Cl.: G01N 35/00, C12Q 1/68, B01L 11/00

(54) **Automatic detection of infectious diseases**

(71) Applicant: Koninklijke Philips Electronics N.V., 5621 BA Eindhoven (NL)
(72) Inventor: The designation of the inventor has not yet been filed
(74) Representative: Rüber, Bernhard Jakob

(57) **Abstract**

The invention relates to the detection of infectious diseases. A system for automatic detection of infectious diseases is disclosed. The system comprises an input unit for receiving a blood sample, a lysis unit, a PCR unit, a sample unit and a detection unit. The system may based on an inputted blood sample generate an output signal that is indicative of the presence of pathogen DNA in the blood sample.

## Description

### FIELD OF THE INVENTION

The invention relates to the detection of infectious diseases, and in particular to a system for detection infection diseases, to a method of operating a system, and to a computer program product.

### BACKGROUND OF THE INVENTION

Patients in intensive care units (ICU) are generally very vulnerable to the development of sepsis. Sepsis is a major cause of death in intensive care units worldwide, and it is of utmost importance to detect it as soon as possible. The cause of sepsis can relate to a variety of different bacteria, vira, funghi or other organisms. The more precisely a certain cause of the sepsis can be attacked by treatment with dedicated antibiotics, the more chance the patient has to recover or even to avoid the development of sepsis. In the event that the origin of the sepsis is not known, broad spectrum antibiotic treatment has to be prescribed, possibly causing the development of resistances or even the death of the patient if the effective antibiotic was not present in the cocktail. The faster the specific type of cause of the infection can be detected, the better the treatment of the infection can be adjusted to inhibit the growth or to destroy the pathogens and avoiding an over-reaction of the patient's immune system.

Detection of the presence of bacteria or other micro-organisms like fungi or viruses takes a long time in standard laboratories, typically a few days. During the time between the taking of the blood sample and the diagnosis of the infectious agent, the patient may be given expensive broad spectrum antibiotic treatment. Furthermore, by transporting samples over different labs and apparatuses, exchange of samples of different patients may happen, leading to false negatives and the sample can be infected otherwise, leading to false positives.

Solutions in the prior art of quickly providing an accurate diagnosis, for example as disclosed in the published application US 2007/0005256, propose to perform real time correlations of data collected from biological sensors. Correlation methods are disclosed. However, in connection with sepsis of the individual patients, at least two types of time delays are of importance, namely the time delay relating to the analysis of the biological sample and the time delay relating to the diagnosis.

The inventors of the present invention have appreciated that improved handling of blood samples in connection with detection of infectious diseases would be of benefit, and have in consequence devised the present invention.

### SUMMARY OF THE INVENTION

The invention preferably seeks to mitigate, alleviate or eliminate one or more of the above mentioned disadvantages singly or in any combination. In particular, it may be seen as an object of the present invention to provide an improved way of handling blood samples in connection with detection of infectious diseases that solves the above mentioned problems, or other problems, of the prior art related to infectious diseases.

This object and several other objects are obtained in a first aspect of the invention by providing a system for automatic detection of infectious diseases, the system comprises:
- an input unit for receiving a blood sample;
- a lysis unit separating DNA content from the received blood sample to a separation sample;
- a PCR unit for receiving the separation sample and multiplying a number of gene sequences in the separation sample to provide a target sample with target molecules;
- a sample unit for receiving the target sample, and contacting the gene sequences with a substrate having immobilized thereon probe molecules that specifically binds the target molecules;
- a detection unit for detecting the presence of resultant binding complexes on the substrate to determine the presence of the target molecules in the blood sample;
wherein an output signal is generated indicative of the presence of predefined target molecules.

The inventors have had the insight that in order to provide a system that is capable of bringing down time delays relating to the detection of an infection in a blood sample, and particularly relating to the detection of a risk of, or a development of, sepsis, a system is needed, which deals with the time delay relating to the analysis of the blood sample.

Embodiments of the first aspect are advantageous for providing a system which facilitates a streamlining of the detection process, thereby rendering it possible to provide a system which is capable of real-time analysis of a blood sample. In this respect it is important to provide a system which avoids or minimizes any delay between obtaining a blood sample and starting the analysis. The system of the present invention enables both direct contact to a blood stream via connection to the input unit, i.e. in-line detection, e.g. by means of direct connection to a catheter, as well as direct input into the input unit of an already obtained blood sample, i.e. at-line detection. In any case, the time-delay between obtaining blood from the patient and starting the analysis is very small or even substantially non-existing. Moreover, by keeping the path short between the blood sampling and the analysis systems, the possibility of blood sample exchange may be avoided or at least kept very small.
Moreover, embodiments of the first aspect are advantageous for providing a complete unit that is capable of detecting the presence of a specific pathogen DNA in a blood sample. The unit may be provided with a size, so that the entire system may be placed in a hospital ward, or even carried by the patient. Sending samples to the laboratory for testing may thereby be avoided.

The system is advantageous in that continuous real time monitoring of severely ill patients prone to sepsis is rendered possible. By continuous monitoring direct information relating to the course of the disease and the effect and efficacy of a prescribed medication can be directly followed.

In the context of the present invention, real time has to be defined in relation to the reaction time of a human body to infections and treatments against that. The time constant of the immune system is of the order of magnitude of hours, so a measurement in the range of 5 to 30 minutes, or even longer, such as up to one hour, can be considered real time.

In an advantageous embodiment, the system may further comprise or be connected to a decision support system. A decision support system may advise the doctor or clinician based on existing knowledge, as well as providing a prediction of a course of the disease. Thereby reducing the time delay involved in obtaining a diagnosis.

In an advantageous embodiment, the decision support system additionally receives a signal indicative of physiological information, such as a signal in the group of: a signal indicative of a temperature, a signal indicative of a blood pressure, a signal indicative of a heart rate, or other relevant signals. By providing also physiological data into the decision support system a broad picture of the course of the disease can be obtained.

In an advantageous embodiment, the decision support system outputs a signal, based on the received inputs, to a medicament administration unit. At least in some situations, the decision support system may be able to make such a precise decision, that it can be used for medicament administration. For example, if a clear indication of an infection is detected, the right medicament may be administered immediately. Fast and automatic adapting of the medication may thereby be made.

In a second aspect, the present invention relates to a method of operating a system for automatic detection of infectious diseases, the method comprises:
- receiving a blood sample;
- separating DNA content from the received blood sample by lysis to provide a separation sample;
- multiplying a number of gene sequences in the separation sample by PCR to provide a target sample with target molecules;
- contacting the target sample with a substrate having immobilized thereon probe molecules that specifically binds the target molecules;
- detecting the presence of resultant binding complexes on the substrate to determine the presence of the target molecules in the blood sample;
and generating a signal indicative of the presence of target molecules.

The method of the third aspect may operate a system in accordance with the first aspect of the invention.

In a third aspect, the present invention relates to a computer program product having a set of instructions, when in use on a computer, to cause a system for automatic detection of infectious diseases to perform the method of the second aspect, or to be implemented in a general or specific purpose computer to operated the system in accordance with the first aspect.
In general, the various aspects of the invention may be combined and coupled in any way possible within the scope of the invention. These and other aspects, features and/or advantages of the invention will be apparent from and elucidated with reference to the embodiments described hereinafter.

### BRIEF DESCRIPTION OF THE DRAWINGS

Embodiments of the invention will be described, by way of example only, with reference to the drawings, in which
- FIG. 1: schematically illustrates an exemplary embodiment of a system for automatic detection of infectious diseases;
- FIG. 2: illustrates an example of a spot pattern printed on a porous substrate; and
- FIG. 3: illustrates a flow diagram of an exemplary embodiment of a method of operating a system for automatic detection of infectious diseases.

### DETAILED DESCRIPTION OF EMBODIMENTS

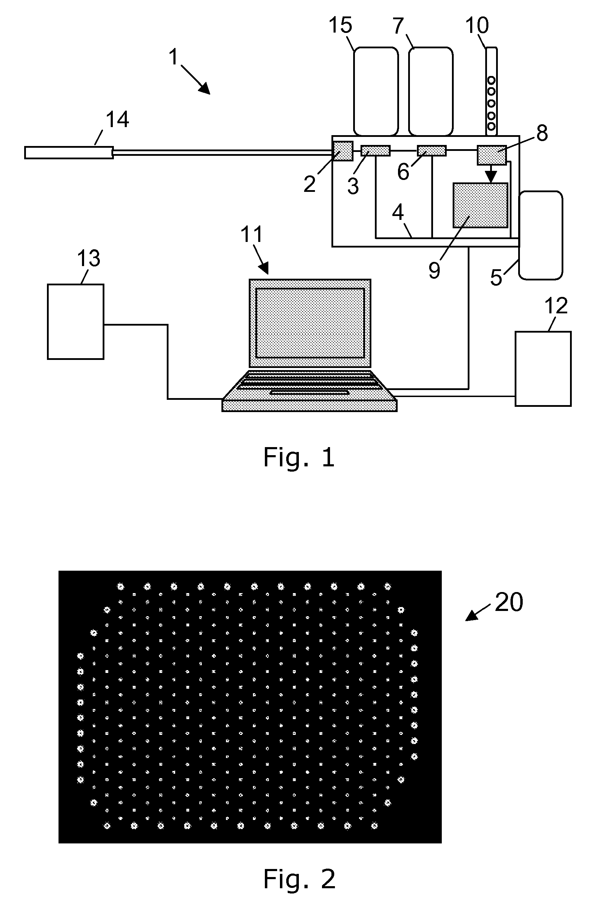

FIG. 1 schematically illustrates an exemplary embodiment of a system 1 for automatic detection of infectious diseases. The detection is done by real time detection of pathogen DNA of bacteria, vira, funghi or other organisms involved in the development of sepsis of a patient.

In the system, a blood sample is provided at an input unit 2. The blood sample may be provided from a catheter 14. In an exemplary embodiment the catheter is placed in a patient under investigation. However, it is to be understood that the operation of the system is not conditioned upon the placement of the catheter in a patient. The blood sample may be provided to the system in any way, including but not limited to, the provision of post-drawn blood samples.

In an exemplary embodiment, around 250 µL (microliters) of blood pr. hour is inputted into the system for analysis. The blood may be provided in a continuous flow, possible in connection with volume units provided at predetermined time intervals, such as 125 µL pr. half hour, 75 µL pr. 15 minutes, or other suitable volume rates. In an embodiment, a continuous flow of blood may be provided by providing volume blocks of blood, separated by a buffer fluid, such as a 150 mM phosphate solution in water or a mixture of water and ethylene glycol or lactated Ringer solution. The throughput of blood may thereby be enhanced. The blood flow may pass through a pump unit that maintains the flow through out the whole instrument.

The blood sample is provided to a lysis unit 3. The blood sample contains a variety of cells such as red blood cells, white blood cells, platelets and possible bacteria, fungi and/or viruses. In an exemplary embodiment, first the red blood cells are disintegrated followed by a disintegration of the cells that contain DNA. In different embodiments, different types of lysis may be applied, either singly or in any combinations. For cells that can easily be disrupted, a mild osmosis-based method may be used, where the ionic strength of the medium is lowered resulting in swelling and bursting of the cells. In other methods, glass or ceramic beads are used with a high level of agitation, different types of mechanical agitation may be applied as well. In such methods, the bulk aqueous media is placed under shear forces that literally pull the cells apart. In yet other methods, detergent-based cell lysis may be applied. The specific detergent used for the cell lysis is selected in dependence on the cell type. Examples of non-ionic detergents include, but are not limited to: CHAPS lysis buffer, a zwitterionic detergent and the Triton X series and SDS lysis buffer. In addition to the choice of detergent, also an appropriate buffer, the pH, the ionic strength and the temperature, may be selected or set appropriately in accordance with the cell type. A specific reagent may be supplied from a lysin fluid reservoir 15 and exposed to the blood sample by microdosing.

After the lysis, the separated DNA may be removed from the lysis solution, e.g. by centrifugation or filtering. Moreover, the DNA content may be washed in continuation of the lysis. Any waste products resulting from the lysis process may be collected into a waste container 5 by means of a fluid system 4. The resulting solution containing the separated DNA content is referred to as the separation sample. A buffer fluid may be added to the separation sample in order to increase the sample volume so as to maintain the flow rate.

The separation sample is provided into a PCR unit 6 for multiplying a number of gene sequences to provide a target sample with target molecules.

In the PCR unit the separation sample is exposed to Polymerase Chain Reaction (PCR). PCR is a technique to exponentially amplify, in vitro, a small quantity of a specific nucleotide sequence, here in terms of the separated gene sequences of the pathogen DNA. In PCR oligonucleotide primers hybridize to the strands of interest in the target pathogen DNA. Primers hybridise at a temperature that is affected by their sequence, concentration, length and ionic environment. By selecting the right primer and a predefined temperature profile during a number of cycles, and other parameters, specific strands of the selected DNA molecules are multiplied, i.e. the gene sequences of the pathogen DNA under investigation are multiplied. The fluids needed for the PCR are contained in a reservoir 7. The PCR process results in a target sample. The multiplied gene sequences are referred to as target molecules.

In an exemplary embodiment, the target molecules are providing with fluorophores, e.g Alexa fluor dyes and Cy dyes. The fluorophores may be provided, by application of primers that contains a fluorophore. Again any waste fluids may be supplied to the reservoir 4, 5.

The PCR unit may in embodiments be operated in accordance with an unsaturated PCR process or a saturated PCR process. A saturated PCR process may be performed in accordance with standard end-point PCR. However, this technique is not entirely quantitative since it works until saturation. As an alternative, an unsaturated PCR process may be made, where a lower number of PCR cycles are run. As an example of an unsaturated PCR process, quantitative real time PCR may be applied.

The target sample is provided to a sample unit 8. In the sample unit, the target sample is contacted with a substrate. Probe molecules are immobilized on the substrate. The probe molecules are selected as molecules that specifically bind or hybridize to the target molecules. In an exemplary embodiment, the contact is provided by flowing the target sample fluid through a substrate in the form of a porous membrane. To increase the efficiency of capturing the target molecules, and thereby increase the detection efficiency, the target sample may be recycled to multiple the passes of the target molecules through the substrate.

In an exemplary embodiment, the substrate or membrane may be in the form of a micro-array. A micro-array can be provided where each spot or group of spots are provided which specifically bind specific target molecules. That is which specifically bind specific stands of DNA. The micro arrays may be mounted in a strip 10 that automatically transport a new array into the instrument when needed. This may be after a fixed time, after detection of one or more intense spots on the micro array, or after saturation in one or more spots have been detected.

In connection with the contacting or possible in continuation of the contacting, washing steps may be performed in order to remove or dilute unbound target molecules.

Binding complexes with specific target molecules will in course of time build up at specific locations on the substrate. Such binding complexes are representative of specific pathogen DNA in the blood sample. From the location, the type of the pathogen DNA can be deduced.

The presence of the binding complexes may be detected in a detection unit 9. The detection unit may either operate on the substrate in the sample unit 8, or the substrate may be transferred to a detection location.

In an exemplary embodiment, the presence of the target molecules is detected by detecting luminescence emanating from the target. The substrate may be illuminated by radiation having characteristics selected in accordance with the fluorophores used. As the DNA strands are provided with fluorophores spots with captured DNA strands will light up. A CCD camera or other optical detector may be used for recording a dot pattern. FIG. 2 provides an example of a dot patterns obtained by a CCD camera. FIG. 2 is discussed in further detail below.

In an exemplary embodiment, the presence of the target molecule is detected by detecting changes in luminescence of the target. By detecting the changes, instead of absolute intensity levels, a larger background luminescence may be tolerated. Moreover, any determination of the absolute level of the background luminescence may be avoided, or at least determined less strictly. The detection of changes in luminescence of the target may be correlated to the amount of pathogen DNA in the patient's blood. The detection of an increase (or decrease) and the increase rate (or decrease rate) may be correlated to a specific disease and possible even to a course of the disease.

In an exemplary embodiment, an output signal may be generated which is indicative of the presence of predefined target molecules. The output signal may be a signal representing the obtained dot pattern. The output signal may be sent to a computing unit 11 for evaluation. In an embodiment, the evaluation provides an output to the user, i.e. the clinical person in charge of the patient of the detected bacterial DNA.

In an exemplary embodiment, the system comprises or is connected to a decision support system, possibly implemented in the computing unit 11. The computing unit 11 and the detection system 1 are illustrated as two separate units, it is however to be understood, that they may be part of a single system.

The decision support system may also be provided with a input signals from physiological measurements 12. The decision support system may thus be provided with input from the detection of the pathogen DNA, i.e. the DNA spectra. The DNA spectra may be time resolved. In addition, the decision support system may be provided with input signals from physiological measurements. Based on such inputs, the decision support system may provide an output which supports a doctor in the task of obtaining a diagnosis. In addition, the decision support system may predict or estimate the course of the disease and provide feedback or guidelines on how to proceed with the treatment.

The system may moreover be connected to a medicament administration unit 13, such as an electronically drip system, a transdermal drug delivery system, an implantable drug delivery device or an electronic pill for controlled drug release. In an exemplary embodiment, the decision support system outputs a signal, based on the received inputs, to the medicament administration unit. In the event that an implantable or swallowable device is used for the administering of the medication, in order to make it adaptable, a wireless link may be used for the communication between the detection system and the devices inside the body. The output signal from the decision support system to the medicament administration unit may be based on a decision of the decision support system, on a decision made by a clinician, or on a combination.

In embodiments of the present invention, time delays are involved in connection with the various aspects between blood extraction and the detection of the pathogen DNA. To bring down the involved time delays, the overall dimension of the device may be small. For example, the overall dimension of the fluid passage between the input unit and the detection unit, is below 50 cm, or even smaller such as 25 cm. Moreover, to increase the fluid flow, a micro fluidic system may be used for transporting the fluid through the device. For example, the cross-sectional area of the flow channels, may be below 0.5 mm² or even smaller such as 0.1 mm². To further increase the fluid flow, suitable buffer fluids may be applied. By application of buffer fluids, flow rates as high as 5×10⁻⁴ m/s or even higher may be obtained. The device may even though not explicitly illustrated comprise one or more pump units or a pumping system, including a number of valves, for ensuring a flow of the fluids through the system.

A further advantage of a small device is that it may be placed close to the patient. The patient may even wear the device.

FIG. 2 illustrates an example of a spot pattern printed on a porous substrate 20. Here it is in the form of a membrane mounted on a supporting structure. The membrane is about 2.4 mm in diameter and covered with a pattern of 392 different capture spots. Each of the spots needs a volume of around 0.1 nL (nanoliter). The diameter of the spots is around 50 µm (micrometers), the spots are placed in a hexagonal pattern with a pitch of 100 µm. It is to be understood, that FIG. 2 mere provides an example substrate, in dependence of the print setup, any suitable substrate may be used.

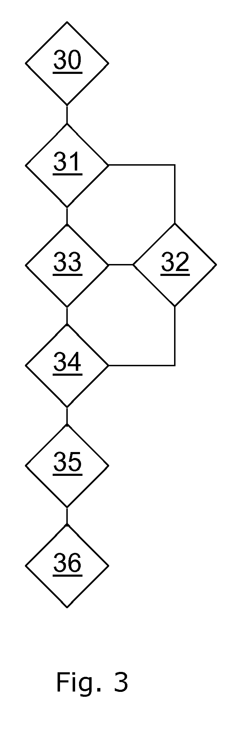

FIG. 3 illustrates a flow diagram of an exemplary embodiment of a method of operating a system for automatic detection of infectious diseases. The method comprises the steps:
30: receive a blood sample;
31: separate the DNA content from the received blood sample by lysis to provide a separation sample. The separation sample is forwarded to a PCR unit, whereas the remaining part may be let 32 to a waste container;
33: multiply a number of gene sequences in the separation sample by PCR to provide a target sample with target molecules, any surplus or waste fluids may be let to the waste container 32;
34: contact the target sample with a substrate having immobilized thereon probe molecules that specifically binds the target molecules, any surplus or waste fluids from the contacting may be let to the waste container 32;
35: detect the presence of resultant binding complexes on the substrate to determine the presence of the target molecules in the blood sample;
36: generate a signal indicative of the presence of target molecules.

The method may for example, be implemented into a control computer, that controls the system as disclosed in connection with FIG. 1, so that an automatic system may be provided.

Although the present invention has been described in connection with the specified embodiments, it is not intended to be limited to the specific form set forth herein. Rather, the scope of the present invention is limited only by the accompanying claims. In the claims, the term "comprising" does not exclude the presence of other elements or steps.

Additionally, although individual features may be included in different claims, these may possibly be advantageously combined, and the inclusion in different claims does not imply that a combination of features is not feasible and/or advantageous. In addition, singular references do not exclude a plurality. Thus, references to "a", "an", "first", "second" etc. do not preclude a plurality. Furthermore, reference signs in the claims shall not be construed as limiting the scope.

## Claims

1. A system for automatic detection of infectious diseases, the system comprises:
- an input unit (2) for receiving a blood sample;
- a lysis unit (3) separating DNA content from the received blood sample to a separation sample;
- a PCR unit (6) for receiving the separation sample and multiplying a number of gene sequences in the separation sample to provide a target sample with target molecules;
- a sample unit (8) for receiving the target sample, and contacting the gene sequences with a substrate (20) having immobilized thereon probe molecules that specifically binds the target molecules;
- a detection unit (9) for detecting the presence of resultant binding complexes on the substrate to determine the presence of the target molecules in the blood sample;
wherein an output signal is generated indicative of the presence of target molecules.

2. The system according to claim 1, wherein the input unit is connected to a catheter (14) for receiving the blood sample.

3. The system according to claim 1 further comprising or being connected to a decision support system, wherein the output signal is inputted into the decision support system.

4. The system according to claim 3, wherein in addition to the output signal, also a signal indicative of physiological information is inputted into the decision support system.

5. The system according to claim 3, wherein the decision support system outputs a signal, based on the received inputs, to a medicament administration unit (13).

6. The system according to claim 1, wherein the input unit provides a continuous flow of the blood sample to the lysis unit or provides a predefined volume of blood at predefined time intervals.

7. The system according to claim 1, wherein the PCR unit is operated in accordance with an unsaturated PCR process or a saturated PCR process.

8. The system according to claim 1, wherein the target molecules are provided with fluorophores.

9. The system according to claim 9, wherein the presence of the target molecules is detected by detecting luminescence of the target.

10. The system according to claim 9, wherein the presence of the target molecule is detected by detecting changes in luminescence of the target.

11. The system according to claim 1, wherein the number of gene sequences is a number of gene sequences of predefined bacteria DNA, funghi DNA or viral DNA.

12. A method of operating a system for automatic detection of infectious diseases, the method comprises:
- receiving (30) a blood sample;
- separating (31) DNA content from the received blood sample by lysis to provide a separation sample;
- multiplying (33) a number of gene sequences in the separation sample by PCR to provide a target sample with target molecules;
- contacting (34) the target sample with a substrate having immobilized thereon probe molecules that specifically binds the target molecules;
- detecting (35) the presence of resultant binding complexes on the substrate to determine the presence of the target molecules in the blood sample; and generating (36) a signal indicative of the presence of target molecules.

13. A computer program product having a set of instructions, when in use on a computer, to cause a system for automatic detection of infectious diseases to perform the steps of claim 12.
